# EUROPÄISCHE PATENTSCHRIFT

(11) **EP 0 839 127 B1**
(45) Veröffentlichungstag und Bekanntmachung des Hinweises auf die Patenterteilung: **31.05.2000**
(21) Anmeldenummer: 96924845.9
(22) Anmeldetag: 02.07.1996
(51) Int. Cl.: C07C 231/02, C07C 233/47, C07C 303/32, C07C 303/22

(54) **VERFAHREN ZUR HERSTELLUNG VON N-ACYLAMINOCARBONSÄUREN UND N-ACYLAMINOSULFONSÄUREN SOWIE DEREN ALKALIMETALLSALZEN**
PROCESS FOR PREPARING N-ACYLAMINOCARBOXYLIC ACIDS AND N-ACYLAMINOSULPHONIC ACIDS AND THE ALKALI METAL SALTS THEREOF
PROCEDE DE PRODUCTION D'ACIDES N-ACYLAMINOCARBOXILIQUES ET D'ACIDES N-ACYLAMINOSULFONIQUES ET LEURS SELS DE METAUX ALCALINS

(30) Priorität: 13.07.1995 DE 19525512
(43) Veröffentlichungstag der Anmeldung: 06.05.1998
(73) Patentinhaber: BASF AKTIENGESELLSCHAFT, 67056 Ludwigshafen (DE)
(72) Erfinder: OFTRING, Alfred, D-67098 Bad Dürkheim (DE); AUS DEM KAHMEN, Martin, D-67071 Ludwigshafen (DE)
(86) Internationale Anmeldenummer: EP9602888
(87) Internationale Veröffentlichungsnummer: WO9703043

(56) Entgegenhaltungen:
- WO-A-95/07881
- DE-A- 4 408 957
- DE-A- 4 433 977
- FR-A- 2 077 090
- PATENT ABSTRACTS OF JAPAN vol. 006, no. 135 (C-115), 22.Juli 1982 & JP,A,57 058653 (SAGAMI CHEM RES CENTER), 8.April 1982,

## Beschreibung

Die vorliegende Erfindung betrifft ein verbessertes Verfahren zur Herstellung von N-Acylaminocarbonsäuren und N-Acylaminosulfonsäure sowie deren Alkalimetallsalzen aus den Alklimetallsalzen von Aminocarbonsäuren bzw. Aminosulfonsäuren und bestimmten Carbonsäureestern.

Aus der WO-A 95/07881 ist bekannt, daß man Alkalimetallsalze von N-Acylsarkosin durch Acylieren von Alkalimetallsarkosinat mit Carbonsäurealkylestern, insbesondere Carbonsäuremethylestern, in Gegenwart von basischen Katalysatoren wie Natriummethylat oder Kaliumethylat bei Temperaturen von 80 bis 200°C herstellen kann. Nachteilig an dieser Methode ist der Einsatz von Carbonsäurealkylestern, z.B. Ölsäuremethylester, welche in einem vorgeschalteten Syntheseschritt aufwendig hergestellt und gegebenenfalls gereinigt werden müssen.

In der JP-A 57-058 653 (Patent Abstracts of Japan, Vol. 6, No. 135 (C-115), 22.07.1982) wird die Herstellung von N-Acylaminosäuren aus Aminosäuren und Fettsäureglyceriden beschrieben.

Aufgabe der vorliegenden Erfindung war es daher, ein effizientes und wirtschaftliches Herstellungsverfahren ausgehend von gut zugänglichen und preiswerten Ausgangsmaterialien bereitzustellen, welches in guten Raum-Zeit-Ausbeuten N-Acylaminocarbonsäuren und N-Acylaminosulfonsäuren bzw. deren Alkalimetallsalze in ausreichenden Reinheiten liefert.

Demgemäß wurde ein Verfahren zur Herstellung von N-Acylaminocarbonsäuren und N-Acylaminosulfonsäuren sowie deren Alkalimetallsalzen aus den Alkalimetallsalzen von Aminocarbonsäuren bzw. Aminosulfonsäuren und Carbonsäureestern gefunden, bei dem man
(a) eine Suspension der festen wasserfreien Alkalimetallsalze der Aminocarbonsäuren bzw. Aminosulfonsäuren in den Carbonsäureestern herstellt,
(b) diese Suspension durch Zusatz von starken Basen zu den Alkalimetallsalzen der N-Acylaminocarbonsäuren bzw. N-Acylaminosulfonsäuren abreagieren läßt und
(c) gewünschtenfalls daraus die freien N-Acylaminocarbonsäuren bzw. N-Acylaminosulfonsäuren in üblicher Weise durch Zugabe von Säuren herstellt
und welches dadurch gekennzeichnet ist, daß man als Carbonsäureester Carbonsäureglyceride in Form von Mono-, Di- oder Triglyceriden von gesättigten oder ungesättigten C₆- bis C₃₀-Monocarbonsäuren oder Mischungen hieraus einsetzt.

Bei Schritt (a) geht man beispielsweise so vor, daß man die Carbonsäureglyceride und die festen wasserfreien Alkalimetallsalze der Aminocarbonsäuren bzw. Aminosulfonsäuren in einem geeigneten Gefäß aus Glas oder einem anderen Material vorlegt und mit einem üblichen suspendierenden Rührgerät zu einer feinteiligen Suspension verarbeitet.

Man kann die Suspension in Schritt (a) aber auch dadurch herstellen, daß man die Carbonsäureglyceride und eine wäßrige Lösung der Alkalimetallsalze der Aminocarbonsäuren bzw. Aminosulfonsäuren in einem geeigneten Gefäß aus Glas oder einem anderen Material miteinander vermischt und anschließend unter Erwärmen und Anlegen eines Vakuums das Wasser möglichst rasch aus dem Gemisch entfernt. Überraschenderweise kann beobachtet werden, daß dabei nur in sehr geringem Umfang eine Verseifung der Carbonsäureglyceride zu den Alkalimetallsalzen der zugrunde liegenden Carbonsäuren auftritt. Der Vorteil der Verwendung von solchen wäßrigen Lösungen ist, daß man im technischen Syntheseprozeß anfallende wäßrige Lösungen von Alkalimetallsalzen von Aminocarbonsäuren bzw. Aminosulfonsäuren ohne weitere Aufbereitung wie Sprühtrocknung einsetzen kann. Außerdem entfallen das Handling mit Feststoffen betreffende Probleme wie Staubbildung oder gleichmäßige Zudosierung.

In der Regel stellt man die Suspension in Schritt (a) aus äquivalenten oder annähernd äquivalenten Mengen von Alkalimetallsalzen von Aminocarbonsäuren bzw. Aminosulfonsäuren, bezogen auf die Acylgruppen in den Carbonsäureglyceriden, und Carbonsäureglyceriden her, also in der Regel im molaren Verhältnis von 3:1 (bei reinen Triglyceriden). Ein Überschuß an Carbonsäureglyceriden, etwa als Verdünnungsmittel, ist normalerweise nicht notwendig. In einigen Fällen kann ein Überschuß an Alkalimetallsalzen von Aminocarbonsäuren bzw. Aminosulfonsäuren, etwa bis einem molaren Verhältnis von 5:1, vorteilhaft sein.

Zu der gemäß Schritt (a) hergestellten Suspension werden in Schritt (b) katalytische, äquimolekulare oder noch höhere Mengen von starken Basen hinzugegeben, um die Umsetzung in Gang zu setzen. Der Zusatz der Basen erfolgt meist nach oder während des Aufheizens der Suspension auf Umsetzungstemperatur, kann aber auch schon bei der Herstellung der Suspension in Schritt (a), beispielsweise zusammen mit den Alkalimetallsalzen der Aminocarbonsäuren bzw. Aminosulfonsäuren, oder kurz vor dem Aufheizen der Suspension erfolgen. Die Basen können als Festsubstanzen oder in gelöster Form, beispielsweise in einem organischen Lösungsmittel wie einem Alkohol, eingesetzt werden.

Die Menge an eingesetzten starken Basen beträgt vorzugsweise 0,5 bis 150 mol-%, insbesondere 1 bis 50 mol-% oder 50 bis 130 mol-%, vorzugsweise 5 bis 20 mol-% oder 70 bis 110 mol-%, bezogen auf die Alkalimetallsalze der Aminocarbonsäuren bzw. Aminosulfonsäuren. Je nach den speziellen Randbedingungen der Umsetzung kann es günstiger sein, katalytische Mengen oder äquimolare Mengen an starken Basen zu verwenden. Man kann eine einzelne Species einer starken Base oder eine Mischung aus verschiedenen Basen verwenden.

Als starke Basen eignen sich insbesondere:
- Alkoholate, vor allem Alkalimetallalkoholate von C₁- bis C₄-Alkanolen, z.B. Natriummethanolat, Natriumethanolat, Natriumisopropylat oder Kalium-tert.-butylat;
- Hydride, z.B. Natriumhydrid, Natriumborhydrid oder Lithiumaluminiumhydrid;
- Alkalimetall- oder Erdalkalimetallhydroxide, z.B. Natriumhydroxid, Kaliumhydroxid, Lithiumhydroxid oder Calciumhydroxid;
- Alkalimetallcarbonate, z.B. Natriumcarbonat, Kaliumcarbonat oder Lithiumcarbonat;
- salzartige Amide, z.B. Lithiumdiisopropylamid;
- Lithiumorganyle wie Alkyllithiumverbindungen, z.B. n-Butyllithium oder Methyllithium, oder Phenyllithium.

### Bevorzugt werden hiervon Alkoholate.

Die Umsetzung in Schritt (b) wird in einem relativ schonenden Temperaturbereich vorgenommen, meist bei 50 bis 200°C, insbesondere bei 80 bis 180°C, vor allem bei 120 bis 160°C. Man arbeitet normalerweise bei Normaldruck; eine Umsetzung unter Eigendruck oder erhöhtem Druck ist zwar möglich, bringt aber keine deutlichen weiteren Vorteile.

Üblicherweise wird nach 1 bis 2 Stunden Umsetzungsdauer nach Basenzugabe im Reaktionsgemisch kein Carbonsäureglycerid mehr durch analytische Methoden, z.B. Infrarot(IR)-Spektroskopie, nachgewiesen. Das bei der Umsetzung aus den Carbonsäureglyceriden freigesetzte Glycerin verbleibt entweder im Reaktionsgemisch oder wird teilweise oder vollständig durch Aufarbeitung nach üblichen Methoden abgetrennt, je nachdem ob es im resultierenden Produkt bei der vorgesehenen Anwendung stört oder nicht stört.

Das Reaktionsgemisch liegt nach beendeter Umsetzung in der Regel als viskose Paste vor. Diese kann nach Absenken der Temperatur, etwa auf 80 bis 100°C, durch Zugabe von Wasser gelöst werden. Man erhält so beispielsweise ca. 30 bis 40 gew.-%ige wäßrige Lösungen der Alkalimetallsalze der N-Acylaminocarbonsäuren bzw. N-Acylaminosulfonsäuren.

Sollen die freien N-Acylaminocarbonsäuren oder N-Acylaminosulfonsäuren erhalten werden, stellt man diese gemäß Schritt (c) aus den Alkalimetallsalzen in üblicher Weise durch Zugabe von Säuren her. Als Säuren eignen sich insbesondere Mineralsäuren wie Schwefelsäure, Phosphorsäure oder Salzsäure, welche meist bei Raumtemperatur zu den wäßrigen Lösungen der Alkalimetallsalze der N-Acylaminocarbonsäuren bzw. N-Acylaminosulfonsäuren gegeben werden, so daß sich ein pH-Wert im Bereich von etwa 0 bis 3, insbesondere 1 bis 2, eingestellt. Dabei entstehen in der Regel milchige cremige Emulsionen. Vorteilhafterweise werden diese Emulsionen mittels eines üblichen Phasentrennhilfsmittels, z.B. Ketonen wie iso-Butylmethylketon oder Methylethylketon, Alkanolen wie n-Butanol, iso-Butanol oder sek.-Butanol, Ethern wie Methyl-tert.-butylether oder Diisopropylether, Essigsäure-C₁- bis C₄-alkylestern, Propionsäure-C₁-bis C₄-alkylestern oder Acetessigester, welches gleichzeitig mit den Säuren oder nach Bildung der Emulsion zugegeben werden kann, bei leicht erhöhter Temperatur, etwa bei 40 bis 70°C, getrennt. Derartige Phasentrennhilfsmittel sind niedrigsiedende, mit Wasser nicht oder wenig mischbare Verbindungen, die kostengünstig großtechnisch einsetzbar sind.

Das erfindungsgemäße Verfahren läßt sich besonders gut anwenden, wenn man als Alkalimetallsalze von Aminocarbonsäuren die Lithium-, insbesondere jedoch die Natrium- oder Kaliumsalze von aliphatischen Aminocarbonsäuren mit 2 bis 10 C-Atomen, vorzugsweise 3 bis 6 C-Atomen, insbesondere von Valin, Leucin, Norleucin, Glycin, Alanin, β-Alanin, ε-Aminocapronsäure, α-Aminoisobuttersäure, Sarkosin (N-Methylglycin), Asparaginsäure, Glutaminsäure oder Iminodiessigsäure einsetzt. Es lassen sich aber auch die Natrium- oder Kaliumsalze von anderen natürlicher. α-Aminosäuren, von Oligopeptiden oder von aromatischen oder cycloaliphatischen Aminocarbonsäuren, z.B. Anthranilsäure, Phenylglycin, Phenylalanin oder 1-Aminocyclohexan-1-carbonsäure, verwenden. Unter Aminocarbonsäuren sind hier vor allem Verbindungen mit einer primären oder sekundären Aminogruppe und einer oder zwei Carboxylgruppen pro Molekül zu verstehen; es können prinzipiell jedoch auch Verbindungen mit mehr als einer Aminogruppe und/oder mehr als zwei Carboxylgruppen eingesetzt werden, die Menge an Carbonsäureglyceriden richtet sich dann nach der Anzahl der Aminogruppen. Alle Carboxylgruppen liegen praktisch vollständig in der Salzform vor.

Das erfindungsgemäße Verfahren läßt sich ebenfalls besonders gut anwenden, wenn man als Alkalimetallsalze von Aminosulfonsäuren die Lithium-, insbesondere jedoch die Natrium- oder Kaliumsalze von aliphatischen Aminosulfonsäuren mit 2 bis 10 C-Atomen, vorzugsweise 2 bis 4 C-Atomen, einsetzt. Insbesondere sind hier die entsprechenden Salze von Taurin (2-Aminoethansulfonsäure) und N-Methyltaurin von Interesse. Genau wie bei den Aminocarbonsäuren können die verwendeten Aminosulfonsäuren, welche ebenfalls praktisch vollständig in der Alkalimetallsalz-Form vorliegen, mehrere Aminogruppen und/oder Sulfonsäuregruppen aufweisen.

Als Carbonsäureglyceride kommen vor allem übliche, natürlich vorkommende Fettsäureglyceride in Betracht. Hierbei handelt es sich meist um Mono-, Di- oder insbesondere Triglyceride von gesättigten oder ungesättigten C₁₀- bis C₂₂-Monocarbonsäuren oder Mischungen hieraus. Beispiele für die zugrunde liegenden Monocarbonsäuren sind Laurinsäure, Myristinsäure, Palmitinsäure, Stearinsäure, Ölsäure, Linolsäure oder Linolensäure. Der Alkohol Glycerin ist in den Glyceriden hierbei mit einer, zwei oder drei dieser Carbonsäuren verestert. In den Di- und Triglycerid-Molekülen können gleiche oder verschiedene Carbonsäuren verestert vorliegen.

In der Natur finden sich diese Carbonsäureglyceride, insbesondere Triglyceride, als pflanzliche oder tierische Fette oder Öle, meist mit einem hohen Anteil an ungesättigten Carbonsäuren, insbesondere an Ölsäure. Vor allem sind hier Sojaöl, Rüböl, Olivenöl, Sonnenblumenöl, Baumwollsaatöl, Erdnußöl, Leinöl, Rapsöl, Kopraöl, Palmfruchtöl, Rizinusöl, Rindertalg oder Fischöl zu nennen. Aber auch Triglyceride mit einem hohen Anteil an gesättigten kürzerkettigen Fettsäuren (z.B. C₁₂, C₁₄) wie Kokosöl oder Palmkernöl lassen sich erfolgreich beim erfindungsgemäßen Verfahren einsetzen.

Mit Hilfe des erfindungsgemäßen Verfahrens lasen sich ausreichend reine N-Acylaminocarbonsäuren und N-Acylaminosulfonsäuren sowie deren Alkalimetallsalze oder Gemische solcher Verbindungen mit unterschiedlichen Acylestern und/oder unterschiedlichen Aminocarbonsäure- bzw. Aminosulfonsäure-Grundkörpern auf einfache und wirtschaftliche Weise herstellen. Derartige Verbindungen eignen sich bekanntermaßen als Emulgatoren oder Tenside auf verschiedensten technischen Gebieten.

Das erfindungsgemäße Verfahren liefert in guten Raum-Zeit-Ausbeuten die gewünschten Produkte ohne vorherige Überführung des Carbonsäureglycerids in einen Carbonsäureniedrigalkylester, z.B. den entsprechenden Methylester. Durch die relativ schonende Temperaturführung treten keine Zersetzungsreaktionen auf, die zu Ausbeuteminderungen und Dunkelfärbungen der Produkte führen. Die Umsetzungen sind in wesentlich kürzerer Zeit vollständig abgelaufen.

Durch den Einsatz von Carbonsäureglyceriden als kostengünstigen und leicht handhabbaren Acylierungskomponenten und die Vermeidung von Salzanfall bei der Acylierung ist das erfindungsgemäße Verfahren großtechnisch höchst attraktiv.

### Beispiel Nr. 1

232,5 g (0,25 mol) Rüböl und 13,5 g (0,075 mol) Natriummethylat (als 30 gew.-%ige Lösung in Methanol) wurden vorgelegt und auf 180°C aufgeheizt. Dann gab man portionsweise unter starkem Rühren 125 g (1,125 mol) Sarkosin-Natrium zu (hergestellt aus einer sprühgetrockneten Sarkosin-Natrium-Lösung in Wasser). Nach vollständiger Zugabe wurde gut verrührt, Wasserstrahlvakuum angelegt und so lange weitergerührt, bis kein Glycerid mehr im Reaktionsgemisch nachgewiesen werden konnte (nach ca. 2 bis 4 Stunden). Anschließend wurde das Reaktionsgemisch auf 80 bis 100°C abgekühlt, mit 700 ml Wasser versetzt und mit konz. Schwefelsäure sauer gestellt (pH 1,5). Nach Zugabe von 240 ml Methylethylketon bildeten sich zwei Phasen aus. Die organische Phase wurde abgetrennt und das Lösungsmittel am Rotationsverdampfer entfernt. Man erhielt 227 g eines gelblichen Öls mit N-Oleoylsarkosinsäure als Hauptbestandteil.

### Beispiel Nr. 2

465 g (0,5 mol) Rüböl und 167 g (1,5 mol) Sarkosin-Natrium wurden zusammengegeben und gut verrührt (Ultra Turax-Rührer). Nach Zugabe von 18 g (0,1 mol) Natriummethylat (als 30 gew.-%ige Lösung in Methanol) wurde vorsichtig auf 140°C erhitzt. Gleichzeitig wurde Wasserstrahlvakuum angelegt. Nach vollständigem Umsatz (ca. 2 bis 5 Stunden) wurde das Reaktionsgemisch auf 80 bis 100°C abgekühlt, mit 700 ml Wasser versetzt und mit konz. Schwefelsäure sauer gestellt (pH 1,5). Nach Zugabe von 240 ml Methylethylketon bildeten sich zwei Phasen aus. Die organische Phase wurde abgetrennt und das Lösungsmittel am Rotationsverdampfer entfernt. Man erhielt 510 g eines gelblichen Öls mit N-Oleoylsarkosinsäure als Hauptbestandteil.

### Beispiel Nr. 3

232,5 g (0,25 mol) Rüböl und 13,5 g (0,075 mol) Natriummethylat (als 30 gew.-%ige Lösung in Methanol) wurden vorgelegt und auf 180°C aufgeheizt. Dann gab man portionsweise unter starkem Rühren 125 g (1,125 mol) Sarkosin-Natrium (hergestellt aus einer sprühgetrockneten Sarkosin-Natrium-Lösung in Wasser) zu. Nach vollständiger Zugabe wurde gut verrührt, Wasserstrahlvakuum angelegt und so lange weitergerührt, bis kein Glycerid mehr im Reaktionsgemisch nachgewiesen werden konnte (nach ca. 2 bis 4 Stunden). Anschließend wurde das Reaktionsgemisch auf 80 bis 100°C abgekühlt, mit 1000 ml Wasser versetzt und solange gerührt, bis sich eine klare wäßrige Lösung gebildet hatte. Die Auswaage betrug 1350 g an N-Oleoylsarkosin-Natrium als Hauptbestandteil enthaltender Lösung.

## Patentansprüche

1. Verfahren zur Herstellung von N-Acylaminocarbonsäuren und N-Acylaminosulfonsäuren sowie deren Alkalimetallsalzen aus den Alkalimetallsalzen von Aminocarbonsäuren bzw. Aminosulfonsäuren und Carbonsäureestern, wobei man
(a) eine Suspension der festen wasserfreien Alkalimetallsalze der Aminocarbonsäuren bzw. Aminosulfonsäuren in den Carbonsäureestern herstellt,
(b) diese Suspension durch Zusatz von starken Basen zu den Alkalimetallsalzen der N-Acylaminocarbonsäuren bzw. N-Acylaminosulfonsäuren abreagieren läßt und
(c) gewünschtenfalls daraus die freien N-Acylaminocarbonsäuren bzw. N-Acylaminosulfonsäuren in üblicher Weise durch Zugabe von Säuren herstellt,
dadurch gekennzeichnet, daß man als Carbonsäureester Carbonsäureglyceride in Form von Mono-, Di- oder Triglyceriden von gesättigten oder ungesättigten C₆- bis C₃₀-Monocarbonsäuren oder Mischungen hieraus einsetzt.

2. Verfahren nach Anspruch 1, wobei man in Schritt (a) eine Suspension aus äquivalenten oder annähernd äquivalenten Mengen von Alkalimetallsalzen von Aminocarbonsäuren bzw. Aminosulfonsäuren, bezogen auf die Acylgruppen in den Carbonsäureglyceriden, und Carbonsäureglyceriden herstellt.

3. Verfahren nach Anspruch 1 oder 2, dadurch gekennzeichnet, daß man in Schritt (b) die starken Basen in Mengen von 0,5 bis 150 mol-%, bezogen auf die Alkalimetallsalze der Aminocarbonsäuren bzw. Aminosulfonsäuren, einsetzt.

4. Verfahren nach den Ansprüchen 1 bis 3, dadurch gekennzeichnet, daß man in Schritt (b) die starken Basen in Mengen von 5 bis 20 mol-% oder 70 bis 110 mol-%, bezogen auf die Alkalimetallsalze der Aminocarbonsäuren bzw. Aminosulfonsäuren, einsetzt.

5. Verfahren nach den Ansprüchen 1 bis 4, wobei man in Schritt (b) Alkalimetallalkoholate als starke Basen einsetzt.

6. Verfahren nach den Ansprüchen 1 bis 5, wobei man die Umsetzung des Schrittes (b) bei Temperaturen von 50 bis 200°C vornimmt.

7. Verfahren nach den Ansprüchen 1 bis 6, wobei man als Alkalimetallsalze von Aminocarbonsäuren die Natrium- oder Kaliumsalze von aliphatischen Aminocarbonsäuren mit 2 bis 10 C-Atomen einsetzt.

8. Verfahren nach den Ansprüchen 1 bis 6, wobei man als Alkalimetallsalze von Aminosulfonsäuren die Natrium- oder Kaliumsalze von aliphatischen Aminosulfonsäuren mit 2 bis 10 C-Atomen einsetzt.

## Claims

1. A process for preparing an N-acylaminocarboxylic or N-acylaminosulfonic acid or an alkali metal salt thereof from an alkali metal aminocarboxylate or aminosulfonate and a carboxylic ester by
(a) preparing a suspension of the solid anhydrous alkali metal aminocarboxylate or aminosulfonate in the carboxylic ester,
(b) adding a strong base to this suspension to form an alkali metal N-acylaminocarboxylate or N-acylaminosulfonate, and
(c) if desired, preparing the free N-acylaminocarboxylic or N-acylaminosulfonic acid therefrom in a conventional manner by adding an acid,
which comprises using a carboxylic ester that is a carboxylic glyceride in the form of a mono-, di- or triglyceride of a saturated or unsaturated monocarboxylic acid having from 6 to 30 carbon atoms or a mixture thereof.

2. A process as claimed in claim 1 wherein, in step (a), a suspension is prepared from equivalent or substantially equivalent amounts of the alkali metal aminocarboxylate or aminosulfonate, based on the acyl groups in the carboxylic glyceride, and of the carboxylic glyceride.

3. A process as claimed in claim 1 or 2 wherein, in step (b), the strong base is used in an amount of from 0.5 to 150 mol%, based on the alkali metal aminocarboxylate or aminosulfonate.

4. A process as claimed in any of claims 1 to 3 wherein, in step (b), the strong base is used in an amount of from 5 to 20 mol% or from 70 to 110 mol%, based on the alkali metal aminocarboxylate or aminosulfonate.

5. A process as claimed in any of claims 1 to 4 wherein, in step (b), the strong base used is an alkali metal alkoxide.

6. A process as claimed in any of claims 1 to 5 wherein the reaction of step (b) is carried out at from 50 to 200°C.

7. A process as claimed in any of claims 1 to 6 wherein the alkali metal aminocarboxylate used is the sodium or potassium salt of an aliphatic aminocarboxylic acid having from 2 to 10 carbon atoms.

8. A process as claimed in any of claims 1 to 6 wherein the alkali metal aminosulfonate used is the sodium or potassium salt of an aliphatic aminosulfonic acid having from 2 to 10 carbon atoms.

## Revendications

1. Procédé pour la production d'acides **N**-acyl-aminocarboxyliques et d'acides **N**-acylaminosulfoniques ainsi que leurs sels de métal alcalin, à partir des sels de métal alcalin d'acides aminocarboxyliques ou aminosulfoniques et d'esters d'acide carboxylique, dans lequel
(a) on prépare une suspension des sels de métal alcalin solides et anhydres et des acides aminocarboxyliques ou des acides aminosulfoniques dans les esters d'acide carboxylique,
(b) on laisse réagir cette suspension en ajoutant des bases fortes pour donner des sels de métal alcalin des acides **N**-acyl-aminocarboxyliques ou des acides **N**-acylamino-sulfoniques et
(c) on prépare, si on le souhaite, à partir de celle-ci les acides **N**-acylaminocarboxyliques ou **N**-acylamino-sulfoniques de façon habituelle en ajoutant des acides,
caractérisé en ce que l'on met en oeuvre en tant qu'esters d'acide carboxylique, des glycérides d'acide carboxylique sous forme de mono-, di- ou triglycérides d'acides monocarboxyliques saturés ou insaturés en C₆ à C₃₀ ou des mélanges de ceux-ci.

2. Procédé selon la revendication 1, dans lequel on prépare dans l'étape (a) une suspension à partir de quantités équivalentes ou approximativement équivalentes de sels de métal alcalin d'acides aminocarboxyliques ou d'acides aminosulfoniques, par rapport aux groupes acyle dans les glycérides d'acide carboxylique, et de glycérides d'acide carboxylique.

3. Procédé selon la revendication 1 ou 2, caractérisé en ce que l'on met en oeuvre dans l'étape (b) les bases fortes en des quantités de 0,5 à 150% en moles, par rapport aux sels de métal alcalin des acides aminocarboxyliques ou des acides aminosulfoniques.

4. Procédé selon les revendications 1 à 3, caractérisé en ce que l'on met en oeuvre dans l'étape (b) les bases fortes en des quantités de 5 à 20% en moles ou de 70 à 110% en moles, par rapport aux sels de métal alcalin des acides aminocarboxyliques ou des acides aminosulfoniques.

5. Procédé selon les revendications 1 à 4, dans lequel l'on met en oeuvre dans l'étape (b) des alcoolates de métal alcalin en tant que bases fortes.

6. Procédé selon les revendications 1 à 5, dans lequel on réalise la réaction de l'étape (b) à des températures de 50 à 200°C.

7. Procédé selon les revendications 1 à 6, dans lequel on met en oeuvre en tant que sels de métal alcalin d'acides aminocarboxyliques, les sels sodiques et potassiques d'acides aminocarboxyliques aliphatiques ayant 2 à 10 atomes de carbone.

8. Procédé selon les revendications 1 à 6, dans lequel on met en oeuvre en tant que sels de métal alcalin d'acides aminosulfoniques, les sels sodiques et potassiques d'acides aminosulfoniques aliphatiques ayant 2 à 10 atomes de carbone.
